# EUROPÄISCHE PATENTSCHRIFT

(11) **EP 2 409 663 B1**
(45) Veröffentlichungstag und Bekanntmachung des Hinweises auf die Patenterteilung: **15.05.2019**
(21) Anmeldenummer: 11173708.6
(22) Anmeldetag: 12.07.2011
(51) Int. Cl.: A61B 50/00

(54) **Behälterunterteil und Behälteroberteil eines Sterilbehälters und Sterilbehälter**
Lower container part and upper container part of a sterile container and sterile container
Parties inférieure et supérieure d'un contenant stérile et contenant stérile

(30) Priorität: 19.07.2010 DE 102010036489
(43) Veröffentlichungstag der Anmeldung: 25.01.2012
(73) Patentinhaber: AESCULAP AG, 78532 Tuttlingen (DE)
(72) Erfinder: Thomas, Stefan, 78532 Tuttlingen (DE)
(74) Vertreter: Hoeger, Stellrecht & Partner Patentanwälte mbB

(56) Entgegenhaltungen:
- DE-U- 1 908 434
- DE-U1-202009 002 968
- DE-U1-202009 004 204

## Beschreibung

Die vorliegende Erfindung betrifft einen Behälterunterteil eines Sterilbehälters, welcher mit einem Behälteroberteil des Sterilbehälters verschließbar ist, welcher Behälterunterteil und welcher Behälteroberteil in einer Schließstellung einen Behälterinnenraum definieren, an welchem Behälterunterteil eine Dichteinrichtung zum gas- oder keimdichten Abdichten des Sterilbehälters in der Schließstellung gehalten ist, welche ein umlaufendes, am Behälterunterteil anliegendes und am Behälteroberteil anlegbares Dichtelement umfasst, wobei das Dichtelement einen Behälterunterteildichtabschnitt mit einem am Behälterunterteil anliegenden Behälterunterteildichtflächenbereich und einen Behälteroberteildichtabschnitt mit einem am Behälteroberteil anlegbaren Behälteroberteildichtflächenbereich umfasst.

Ferner betrifft die vorliegende Erfindung einen Behälteroberteil eines Sterilbehälters, welcher Behälteroberteil ausgebildet ist zum Verschließen eines Behälterunterteils des Sterilbehälters, welcher Behälteroberteil und welcher Behälterunterteil in einer Schließstellung einen Behälterinnenraum definieren, an welchem Behälteroberteil eine Dichteinrichtung zum gas- oder keimdichten Abdichten des Sterilbehälters in der Schließstellung gehalten ist, welche ein umlaufendes, am Behälteroberteil anliegendes und am Behälterunterteil anlegbares Dichtelement umfasst, wobei das Dichtelement einen Behälteroberteildichtabschnitt mit einem am Behälteroberteil anliegenden Behälteroberteildichtflächenbereich und einen Behälterunterteildichtabschnitt mit einem am Behälterunterteil anlegbaren Behälterunterteildichtflächenbereich umfasst.

Des Weiteren betrifft die vorliegende Erfindung einen Sterilbehälter mit einem Behälterunterteil und einem Behälteroberteil zum Verschließen des Behälterunterteils, welcher Behälterunterteil und welcher Behälteroberteil in einer Schließstellung einen Behälterinnenraum definieren, wobei eine Dichteinrichtung zum gas- oder keimdichten Abdichten des Sterilbehälters in der Schließstellung vorgesehen ist, welche ein umlaufendes und sowohl am Behälterunterteil als auch am Behälteroberteil in der Schließstellung anliegendes Dichtelement umfasst, wobei das Dichtelement einen Behälteroberteildichtabschnitt mit einem am Behälteroberteil anliegenden Behälteroberteildichtflächenbereich und einen Behälterunterteildichtabschnitt mit einem am Behälterunterteil anliegenden Behälterunterteildichtflächenbereich umfasst.

Sterilbehälter, die auch als Sterilcontainer bezeichnet werden, mit Behälterunterteilen und Behälteroberteilen der eingangs beschriebenen Art sind in vielfältiger Weise bekannt. Sie dienen in bekannter Weise insbesondere zur keimfreien Lagerung zuvor gereinigter und sterilisierter chirurgischer Instrumente und Implantate. Als Dichtelemente sind hauptsächlich Dichtungsschnüre, Dichtlippen und ähnliche Dichtelemente aus Silikon, Silikonschäumen und ähnlichen Materialien im Einsatz. Ein Problem dieser bekannten Dichtelemente ist deren teilweise nur geringe Kompressionsvariabilität, durch die eine Abdichtung nur in einem kleinen, definierten Abstandsbereich der gegeneinander abzudichtenden Geometrien von Behälterunterteil und Behälteroberteil sichergestellt ist.

Sterilbehälter im dauerhaften Einsatz altern. Zum einen betroffen von Alterungsprozessen ist das Dichtelement selbst, das insbesondere abgenutzt werden und seine Elastizität teilweise oder ganz verlieren kann. Ferner können sich Behälterunterteile definierende Wannen oder Behälteroberteile definierende Deckel der Sterilbehälter verziehen oder durch Stöße und Schläge verbogen werden. Dadurch sind die ursprünglich definierten Abstände im Bereich des Dichtelements zwischen Behälteroberteil und Behälterunterteil verändert. Je nach verwendetem Dichtelement können dann Undichtigkeiten am Behälter entstehen, so dass eine keimdichte Abdichtung des Sterilbehälters nicht mehr gewährleistet ist.

Ferner führt die Alterung von gummielastischen Dichtungsmaterialien zu einer zeitlichen Einschränkung der Anwendung derartiger bekannter Dichtelemente, und zwar mit der Folge, dass der Zustand des Dichtelements nach einem mehrjährigen Einsatz des Sterilbehälters nicht sicher oder nur sehr schwer eingeschätzt werden kann.

Sterilbehälter, insbesondere Unter- und Oberteile derselben, sind beispielsweise aus der DE 20 2009 002 968 U1 und aus der DE 20 2009 004 204 U1 bekannt. In der DE 19 08 434 U sind Schraubkappen mit eingelegtem Dichtungsring für den flüssigkeitsdichten Verschluss eines Füll- und Ausgießstutzens beschrieben.

Es ist daher Aufgabe der vorliegenden Erfindung, ein Behälterunterteil, ein Behälteroberteil und einen Sterilbehälter der eingangs beschriebenen Art so zu verbessern, dass eine keimdichte Abdichtung des Sterilbehälters in der Schließstellung dauerhaft erreicht werden kann.

Diese Aufgabe wird erfindungsgemäß durch ein Behälterunterteil mit den Merkmalen des Anspruchs 1 gelöst .

Eine solche Vorspanneinrichtung vorzusehen stellt insbesondere sicher, dass das Dichtelement in definierter Weise sowohl gegen das Behälterunterteil als auch gegen ein Behälteroberteil in der Schließstellung gedrückt wird. Altert das Dichtelement und verliert es zunehmend seine ursprünglich elastischen Eigenschaften, so spielt dies bei einem erfindungsgemäß vorgeschlagenen Behälterunterteil praktisch keine Rolle, da die Vorspanneinrichtung die Elastizität der Dichteinrichtung im Wesentlichen dauerhaft definiert. Das Dichtelement selbst benötigt nur noch eine geringe Kompressionsvariabilität, da diese von der Vorspanneinrichtung übernommen wird. Auf diese Weise kann eine deutlich längere Standzeit von Sterilbehältern erreicht werden als dies bei herkömmlichen Sterilbehältern der Fall ist. Die klassische Dichtungsfunktion der Dichteinrichtung wird bei dem vorgeschlagenen Behälterunterteil im Wesentlichen getrennt in zwei zum einen von der Vorspanneinrichtung und zum anderen vom Dichtelement ausgeübte Funktionen. Die Vorspanneinrichtung hat überwiegend eine federnde Wirkung und die Dichtigkeit kann durch das Dichtelement erreicht werden. Durch entsprechende Wahl der Vorspanneinrichtung und des Dichtelements können so gezielt ganz Kompressibilitäts- und Dichtigkeitseigenschaften der Dichteinrichtung optimiert werden, da insbesondere aus zwei unterschiedlichen Materialien hergestellte Dichteinrichtungen möglich sind. Der Aufbau der Vorspanneinrichtung wird dadurch besonders einfach, dass diese mindestens ein Vorspannglied umfasst, welches eine vorspannende Kraft auf mindestens den Behälterunterteildichtabschnitt in Richtung auf den Behälterunterteil oder den Behälteroberteildichtabschnitt in Richtung auf den Behälteroberteil ausübt. Das mindestens eine Vorspannglied dient somit dazu, zur Abdichtung von Behälteroberteil und Behälterunterteil vorgesehene Teilbereiche des Dichtelements gegen den Behälteroberteil beziehungsweise den Behälterunterteil zu drücken. Ein besonders einfacher Aufbau der Dichteinrichtung wird insbesondere dadurch erreicht, dass das mindestens eine Vorspannglied eine Schraubenfeder umfasst oder in Form einer Schraubenfeder ausgebildet ist. Schraubenfedern sind einfach herzustellen, insbesondere auch mit genau definiert einstellbaren Federkonstanten, beispielsweise durch entsprechende Auswahl des Materials und abhängig von einer Stärke des Materials, aus dem die Schraubenfeder gewickelt ist. Die Schraubenfeder ist vorzugsweise schräg bezogen auf eine von ihr definierte Schraubenlinie gewickelt, so dass einzelne Windungen der Schraubenfeder nicht im Wesentlichen in einer Ebene senkrecht zur Schraubenlinie verlaufen, sondern relativ zu dieser bereits in einer Grundstellung der Schraubenfeder, in welcher keine äußeren Kräfte auf diese einwirken, um einen Neigungswinkel, beispielsweise um 45°, geneigt sind. Man kann in diesem Fall auch sagen, dass die die Schraubenfeder definierenden Windungen zur Schraubenlinie oder Schraubenachse verkippt liegen. Gemäß der Erfindung ist vorgesehen, dass die Schraubenfeder eine umlaufende Schraubenlinie definiert und dass die Schraubenfeder eine quer, insbesondere senkrecht, zur Vorspanngliedlängslinie wirkende Kraft auf den Behälteroberteildichtabschnitt und/oder den Behälterunterteildichtabschnitt ausübt. Die Schraubenfeder übt somit nicht längs ihrer Vorspanngliedlängslinie eine Kraft auf das Dichtelement aus, was bei Druck- oder Zugfedern typischerweise der Fall ist, sondern gerade senkrecht zur Schraubenlinie. Die Schraubenlinie bildet diejenige Linie, um die sich die Windungen der Schraubenfeder herum erstrecken. Sie verläuft somit in der Regel vollständig im Inneren der Schraubenfeder.

Die gestellte Aufgabe wird ferner durch ein Behälteroberteil mit den Merkmalen des Anspruchs 2 gelöst .

Die oben in Verbindung mit dem erfindungsgemäß verbesserten Behälterunterteil beschriebenen Vorteile weist das erfindungsgemäß vorgeschlagene Behälteroberteil in analoger Weise auf.

Des Weiteren wird die gestellte Aufgabe durch einen Sterilbehälter mit den Merkmalen des Anspruchs 3 gelöst .

Wie bereits in Verbindung mit dem verbesserten Behälterunterteil dargelegt, lassen sich Eigenschaften der Dichteinrichtung durch Trennung der Funktionen Vorspannung/Elastizität/Kompressibilität und Abdichtung verbessern. Mit einer Vorspanneinrichtung, die praktisch keine Alterung zeigt, kann die Dichteinrichtung insgesamt eine sehr lange Standzeit aufweisen, auch wenn das Dichtelement selbst im Laufe der Zeit seine elastischen Eigenschaften zunehmend verliert. Insgesamt können so Sterilbehälter realisiert werden, die im Vergleich zu herkömmlichen Sterilbehältern deutliche längere Standzeiten aufweisen, da die verbesserte Dichteinrichtung eine gegenüber herkömmlichen Dichtungen, beispielsweise Silikonprofilen, eine deutlich verlängerte Lebensdauer aufweist. Der Aufbau der Vorspanneinrichtung wird dadurch besonders einfach, dass diese mindestens ein Vorspannglied umfasst, welches eine vorspannende Kraft auf mindestens den Behälterunterteildichtabschnitt in Richtung auf den Behälterunterteil oder den Behälteroberteildichtabschnitt in Richtung auf den Behälteroberteil ausübt. Das mindestens eine Vorspannglied dient somit dazu, zur Abdichtung von Behälteroberteil und Behälterunterteil vorgesehene Teilbereiche des Dichtelements gegen den Behälteroberteil beziehungsweise den Behälterunterteil zu drücken. Ein besonders einfacher Aufbau der Dichteinrichtung wird insbesondere dadurch erreicht, dass das mindestens eine Vorspannglied eine Schraubenfeder umfasst oder in Form einer Schraubenfeder ausgebildet ist. Schraubenfedern sind einfach herzustellen, insbesondere auch mit genau definiert einstellbaren Federkonstanten, beispielsweise durch entsprechende Auswahl des Materials und abhängig von einer Stärke des Materials, aus dem die Schraubenfeder gewickelt ist. Die Schraubenfeder ist vorzugsweise schräg bezogen auf eine von ihr definierte Schraubenlinie gewickelt, so dass einzelne Windungen der Schraubenfeder nicht im Wesentlichen in einer Ebene senkrecht zur Schraubenlinie verlaufen, sondern relativ zu dieser bereits in einer Grundstellung der Schraubenfeder, in welcher keine äußeren Kräfte auf diese einwirken, um einen Neigungswinkel, beispielsweise um 45°, geneigt sind. Man kann in diesem Fall auch sagen, dass die die Schraubenfeder definierenden Windungen zur Schraubenlinie oder Schraubenachse verkippt liegen. Gemäß der Erfindung ist vorgesehen, dass die Schraubenfeder eine umlaufende Schraubenlinie definiert und dass die Schraubenfeder eine quer, insbesondere senkrecht, zur Vorspanngliedlängslinie wirkende Kraft auf den Behälteroberteildichtabschnitt und/oder den Behälterunterteildichtabschnitt ausübt. Die Schraubenfeder übt somit nicht längs ihrer Vorspanngliedlängslinie eine Kraft auf das Dichtelement aus, was bei Druck- oder Zugfedern typischerweise der Fall ist, sondern gerade senkrecht zur Schraubenlinie. Die Schraubenlinie bildet diejenige Linie, um die sich die Windungen der Schraubenfeder herum erstrecken. Sie verläuft somit in der Regel vollständig im Inneren der Schraubenfeder.

Eine besonders sichere Abdichtung zwischen Behälterunterteil und Behälteroberteil in der Schließstellung kann insbesondere dadurch erreicht werden, dass die Vorspanneinrichtung zum Ausüben einer vorspannenden Kraft auf den Behälterunterteildichtabschnitt und den Behälteroberteildichtabschnitt angeordnet ist. Dies gestattet es, mit einer einzigen Vorspanneinrichtung das Dichtelement so gegen den Behälterunterteil und den Behälteroberteil zu drücken, dass der Behälterinnenraum dauerhaft keimdicht verschlossen werden kann.

Die Keimdichtheit in der Schließstellung eines Sterilbehälters kann weiter verbessert werden, wenn der Behälterunterteildichtabschnitt und der Behälterunterteil relativ zueinander bewegbar angeordnet sind und/oder wenn der Behälteroberteildichtabschnitt und der Behälteroberteil relativ zueinander bewegbar angeordnet sind. Auf diese Weise können eventuell durch Beschädigungen entstandene Verformungen am Behälterunterteil beziehungsweise am Behälteroberteil durch die Dichteinrichtung ausgeglichen werden, ohne dass das Risiko besteht, dass die erforderliche Keimdichtheit des Sterilbehälters nicht mehr gewährleistet ist.

Um zu verhindern, dass sich die Dichteinrichtung in unerwünschter Weise vom Sterilbehälter oder einem Teil desselben lösen kann, ist es vorteilhaft, wenn der Behälterunterteildichtabschnitt fest mit dem Behälterunterteil oder der Behälteroberteildichtabschnitt fest mit dem Behälteroberteil verbunden ist. Mit anderen Worten kann die Dichteinrichtung beispielsweise am Behälterunterteil oder am Behälteroberteil festgelegt sein, und dann mit dem jeweils anderen Teil beim Schließen des Sterilbehälters zusammenzuwirken um den Behälterinnenraum abzudichten.

Die Dichteinrichtung kann grundsätzlich lösbar verbindbar mit dem Behälterunterteil oder dem Behälteroberteil ausgebildet sein. Um dauerhaft zu vermeiden, dass sich die Dichteinrichtung vom Behälterunterteil oder vom Behälteroberteil des Sterilbehälters lösen kann, ist es günstig, wenn der Behälterunterteildichtabschnitt unlösbar mit dem Behälterunterteil oder der Behälteroberteildichtabschnitt unlösbar mit dem Behälteroberteil verbunden ist.

Eine besonders gute Abdichtung kann insbesondere dadurch erreicht werden, dass das Dichtelement einstückig ausgebildet ist. So ergeben sich bei einer umlaufenden Dichtfläche am Behälterunterteil und am Behälteroberteil keine zusätzlichen Spalte zwischen einem Anfang und einem Ende des Dichtelements.

Vorteilhaft ist es, wenn das Dichtelement schlauch- oder röhrenförmig ausgebildet ist. Dies ermöglicht es insbesondere, die Vorspanneinrichtung in einen vom Dichtelement definierten Innenraum anzuordnen, wodurch das Dichtelement quasi eine Hülle für die Vorspanneinrichtung bildet und diese beispielsweise auch gegen Alterung oder Korrosion schützen kann.

Vorzugsweise definiert das Dichtelement einen umlaufenden Hohlraum. Dieser kann einerseits zur Aufnahme der Vorspanneinrichtung dienen. Zum anderen kann er auch Teil einer zusätzlichen Dichtlippe sein, die am Behälteroberteil oder am Behälterunterteil anliegt.

Um auf einfache Weise sicherzustellen, dass die Vorspanneinrichtung eine vorspannende Wirkung auf das Dichtelement, insbesondere Teile desselben, ausüben kann, ist es vorteilhaft, wenn die Vorspanneinrichtung mindestens teilweise vom Dichtelement bedeckt oder umgeben ist. Insbesondere kann die Vorspanneinrichtung derart angeordnet sein, dass der Behälteroberteildichtabschnitt und der Behälterunterteildichtabschnitt in der Schließstellung gegen dafür vorgesehene Anlageflächen des Behälteroberteils und des Behälterunterteils gedrückt werden.

Ein besonders guter Schutz der Vorspanneinrichtung kann insbesondere dadurch erreicht werden, dass das Dichtelement die Vorspanneinrichtung vollständig umhüllt.

Grundsätzlich wäre es möglich, zwei oder mehr Vorspannglieder vorzusehen, die dann gemeinsam die Vorspanneinrichtung ausbilden. Die Konstruktion und die Herstellung des Sterilbehälters sowie der diesen ausbildenden Teile lassen sich weiter vereinfachen, wenn nur ein einziges in sich geschlossen ausgebildetes Vorspannglied vorgesehen ist. Dieses lässt sich beispielsweise einfach in eine dafür vorgesehene Ausnehmung des Dichtelements einschieben.

Gemäß einer weiteren bevorzugten Ausführungsform der Erfindung kann vorgesehen sein, dass das mindestens eine Vorspannglied eine Vorspanngliedlängslinie definiert und dass das mindestens eine Vorspannglied mit einer quer, insbesondere senkrecht, zur Vorspanngliedlängslinie orientierten Außenfläche am Behälteroberteildichtabschnitt und/oder am Behälterunterteildichtabschnitt anliegt. Das Besondere bei dieser Ausgestaltung ist somit, dass das mindestens eine Vorspannglied nicht in Richtung seiner Vorspanngliedlängslinie wirkt, beispielsweise einer Schraubenachse oder -linie bei einer Spiralfeder, sondern gerade in einer Richtung quer zu dieser Vorspanngliedlängslinie. Dies hat jedoch den Vorteil, dass sich aufgrund einer elastischen Verformung des Vorspannglieds praktisch keine Längenänderung desselben in Richtung der Vorspanngliedlängslinie ergibt. Eine Elastizität des mindestens einen Vorspannglieds kann so insbesondere durch die Wahl und Stärke des zur Ausbildung des mindestens einen Vorspannglieds verwendeten Materials eingestellt werden.

Günstig kann es ferner sein, wenn das mindestens eine Vorspannglied eine Vorspanngliedlängslinie definiert und wenn das mindestens eine Vorspannglied eine quer, insbesondere senkrecht, zur Vorspanngliedlängslinie wirkende Kraft auf den Behälteroberteildichtabschnitt und/oder den Behälterunterteildichtabschnitt ausübt. Das Besondere auch bei dieser Ausgestaltung ist somit, dass das mindestens eine Vorspannglied nicht in Richtung seiner Vorspanngliedlängslinie wirkt, beispielsweise einer Schraubenachse oder -linie bei einer Spiralfeder, sondern gerade in einer Richtung quer zu dieser Vorspanngliedlängslinie.

Beschädigungen des Dichtelements durch das mindestens eine Vorspannglied können insbesondere auf einfache Weise dadurch vermieden werden, dass die Schraubenfeder in sich geschlossen ausgebildet ist. Sie weist dann keinen Anfang und kein Ende auf und somit auch keine scharfen Ecken und Kanten, wenn sie aus einem im Querschnitt kreis- oder ovalförmigen Drahtmaterial gebogen ist.

Die Herstellung des Dichtelements lässt sich weiter vereinfachen, wenn es in Form eines Profilstranges ausgebildet ist. Es lässt sich so beispielsweise durch Spritzgießen oder Extrudieren aus einem dafür geeigneten Kunststoffmaterial, beispielsweise einem Elastomer oder einem Kunststoff mit zumindest teilweise elastomeren Eigenschaften, herstellen.

Vorzugsweise umfasst das Dichtelement eine Vorspanneinrichtungsaufnahme zum mindestens teilweisen Aufnehmen der Vorspanneinrichtung. Zur Vorbereitung beziehungsweise zur Montage der Dichteinrichtung kann dann die Vorspanneinrichtung ganz oder teilweise in die Vorspanneinrichtungsaufnahme eingeführt werden.

Vorteilhaft ist es, wenn die Vorspanneinrichtungsaufnahme umlaufend geöffnet ist. Dies gestattet es, das Dichtelement einstückig und in sich geschlossen auszubilden und ein ebenfalls einstückig in sich geschlossen ausgebildetes Vorspannglied seitlich in die Vorspanneinrichtungsaufnahme einzusetzen. Auf diese Weise können nahtlos ausgebildete Dichtelemente und Vorspannglieder einfach und sicher miteinander verbunden werden.

Alternativ kann es auch vorteilhaft sein, wenn die Vorspanneinrichtungsaufnahme geschlossen ist. Dies ermöglicht es, das Vorspannglied beziehungsweise die Vorspanneinrichtung vollständig im beispielsweise einen umlaufenden Hohlraum definierenden Inneren des Dichtelements in der Vorspanneinrichtungsaufnahme anzuordnen, so dass das Dichtelement die Vorspanneinrichtung beispielsweise gegen Alterung oder Korrosion schützen kann.

Günstig ist es, wenn die Dichtungseinrichtung in einer am Behälteroberteil oder am Behälterunterteil umlaufenden Nut angeordnet ist. Die Dichtungseinrichtung lässt sich so auf einfache Weise sicher festlegen. Beispielsweise kann am jeweils anderen Behälterteil ein in die Nut eingreifender Vorsprung ausgebildet sein, der in der Schließstellung gegen die Dichtungseinrichtung drückt.

Um das Risiko zu minimieren, dass sich die Vorspanneinrichtung in unerwünschter Weise vom Behälterunterteil, vom Behälteroberteil oder vom Sterilbehälter löst, ist es günstig, wenn die Vorspanneinrichtung am Dichtelement gehalten ist. Ist das Dichtelement sicher am Sterilbehälter oder an einem Teil desselben gehalten, ist damit automatisch auch die Vorspanneinrichtung in gewünschter Weise gehalten und positioniert.

Um die Standzeit des Sterilbehälters und der ihn ausbildenden Teile zu erhöhen, ist es vorteilhaft, wenn die Vorspanneinrichtung mindestens teilweise aus einem Metall hergestellt ist. Insbesondere lassen sich so dauerhaft und gezielt gewünschte Vorspannkräfte, die durch die Vorspanneinrichtung ausgeübt werden sollen, definieren und konservieren.

Besonders gut geeignet sind Metalle, die Kupfer und/oder Beryllium enthalten. Mit ihnen lassen sich gewünschte Federkonstanten optimal realisieren.

Die Herstellung der Dichteinrichtung wird vereinfacht sowie die Kosten hierfür minimiert, wenn das Dichtelement aus einem Kunststoff hergestellt ist.

Besonders gute Abdichtungsergebnisse lassen sich erzielen, wenn der Kunststoff Gummi und/oder Polytetrafluorethylen enthält.

Die nachfolgende Beschreibung bevorzugten Ausführungsformen der Erfindung dient im Zusammenhang mit der Zeichnung der näheren Erläuterung. Es zeigen:
- Figur 1:: eine schematische perspektivische Ansicht eines ersten Ausführungsbeispiels eines Sterilbehälters mit einem Behälterunterteil und einem Behälteroberteil;
- Figur 2:: eine Schnittansicht längs Linie 2-2 in Figur 1 bei geschlossenem, keimdichten Sterilbehälter;
- Figur 3:: eine Schnittansicht längs Linie 2-2 beim Abnehmen des Behälteroberteils vom Behälterunterteil;
- Figur 4:: eine schematische perspektivische Gesamtansicht einer alternativen Ausführungsform eines Sterilbehälters;

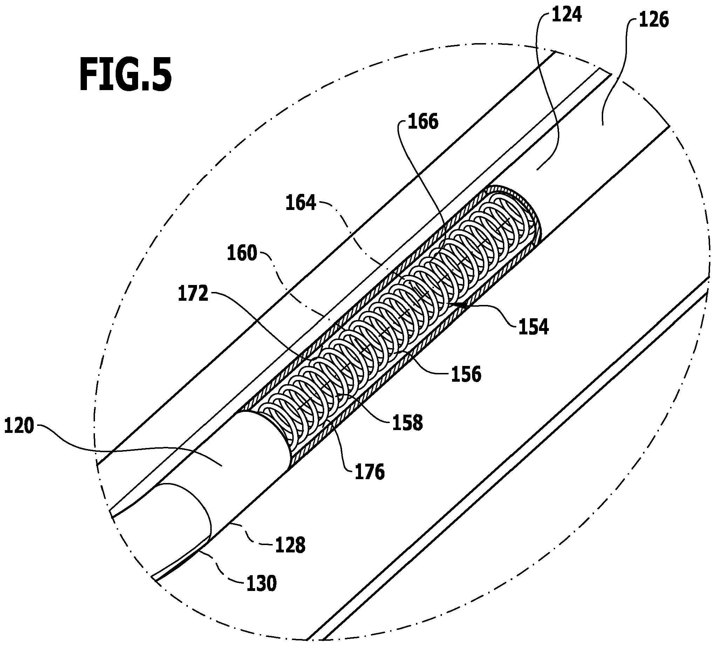

- Figur 5:: eine vergrößerte und teilweise durchbrochene Ansicht des Bereichs A in Figur 4;
- Figur 6:: eine schematische Darstellung der Vorspanneinrichtung in einer Grundstellung;
- Figur 7:: eine Ansicht analog Figur 6 mit teilweise vorgespanntem Vorspannglied; und
- Figur 8:: eine Ansicht analog Figur 6 bei maximal vorgespanntem Vorspannglied.

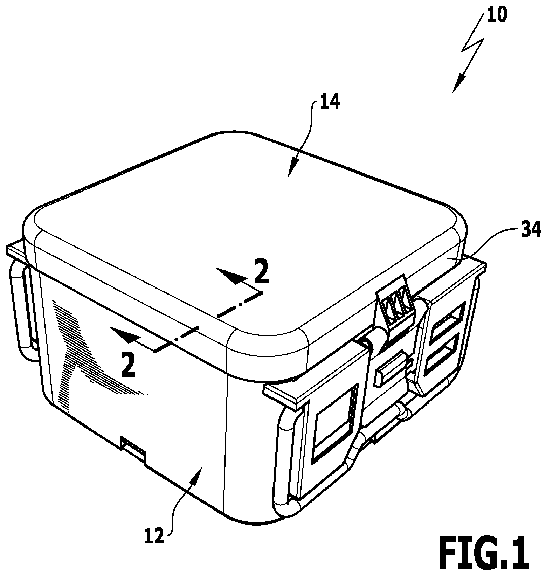

In Figur 1 ist ein insgesamt mit dem Bezugszeichen 10 versehener Sterilbehälter, der auch als Sterilcontainer bezeichnet werden kann, schematisch dargestellt. Er umfasst ein wannenförmiges Behälterunterteil 12 und ein deckelförmiges Behälteroberteil 14 zum Verschließen des Behälterunterteils 12. Wände des Behälterunterteils 12 und des Behälteroberteils 14 begrenzen in einer in Figur 1 dargestellten Schließstellung des Sterilbehälters 10 einen Behälterinnenraum 16.

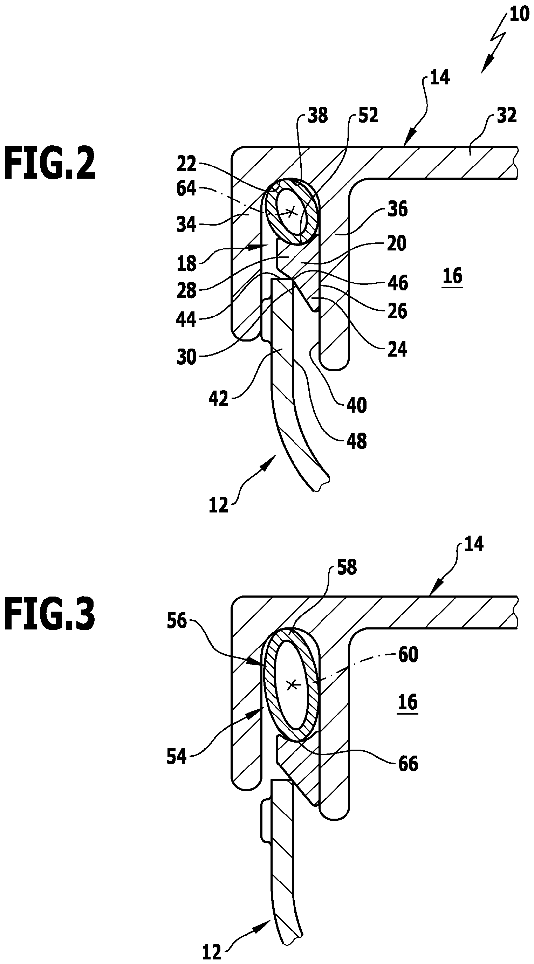

Zum Abdichten des Behälterinnenraums 16 in der Schließstellung dient eine Dichteinrichtung 18. Sie umfasst ein umlaufendes Dichtelement 20, das am Behälterunterteil 12 oder alternativ am Behälteroberteil 14, wie schematisch in den Figuren 2 und 3 dargestellt, in einer am Behälteroberteil 14 umlaufenden Nut 22 angeordnet ist. Alternativ könnte auch am Behälterunterteil 12 eine entsprechende Nut zur Aufnahme der Dichteinrichtung 18 vorgesehen sein.

Das Dichtelement 20 weist einen Behälteroberteildichtabschnitt 24 mit einem am Behälteroberteil 14 anliegenden Behälteroberteildichtflächenbereich 26 auf. Ferner umfasst das Dichtelement 20 einen Behälterunterteildichtabschnitt 28 mit einem am Behälterunterteil 12 anliegenden Behälterunterteildichtflächenbereich 30.

Die Nut 22 wird definiert durch zwei parallel zueinander, von einer Deckelwand 32 senkrecht abstehende Seitenwände 34 und 36, wobei die Seitenwand 34 das Behälteroberteil 14 außen seitlich begrenzt und etwas kürzer ist als die von ihr etwas beabstandete Seitenwand 36. Ein Nutboden 38 ist im Querschnitt halbkreisförmig ausgebildet und in Richtung auf das Behälterunterteil 12 hin weisend konkav gekrümmt.

Der Behälteroberteildichtflächenbereich 26 ist eben ausgebildet und liegt flächig an einer in Richtung auf die Seitenwand 34 weisenden Wandfläche 40 der Seitenwand 36 an. Ein in Richtung auf das Behälteroberteil 14 weisender Endabschnitt 42 des Behälterunterteils 12 ist wandförmig ausgebildet und weist eine in Richtung auf den Nutboden 38 hin weisende, ebene Stirnfläche 44 auf. Eine umlaufende Längskante 46, die zwischen der Stirnfläche 44 und einer auf die Wandfläche 40 hin weisenden Innenfläche 48 des Endabschnitts 42 definiert wird, liegt am in Richtung auf das Behälterunterteil 12 hin weisend, schwach konkav gekrümmten Behälterunterteildichtflächenbereich 30 an.

Das Dichtelement 20 weist eine in Richtung auf den Nutboden 38 hin weisende, in einer Grundstellung, wie schematisch in Figur 3 dargestellt, ebene und in einer Schließstellung, wie schematisch in Figur 2 dargestellt, schwach konvex gekrümmte Anlagefläche 52 auf.

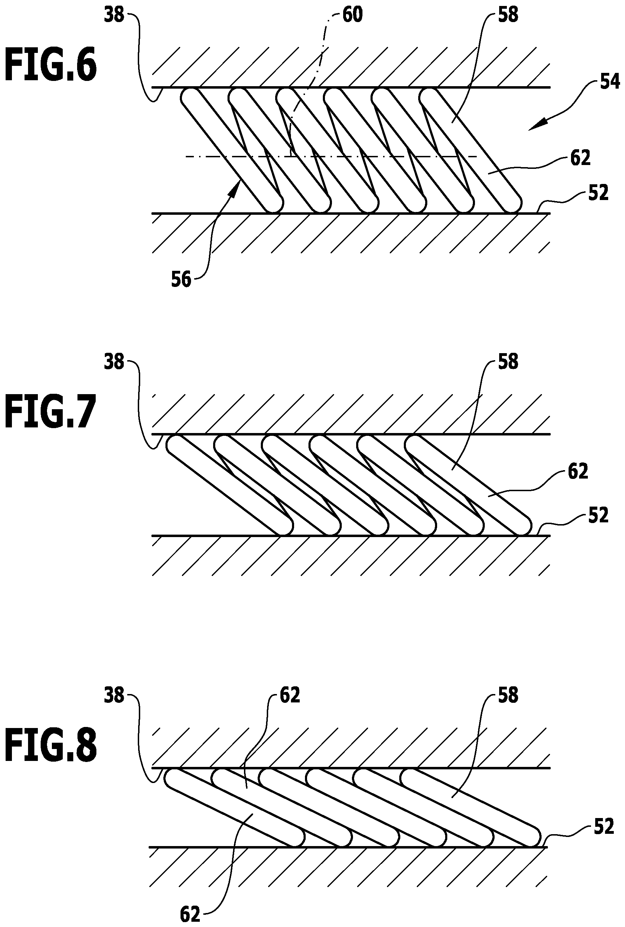

Die Dichteinrichtung 18 umfasst ferner eine Vorspanneinrichtung 54, welche ein Vorspannglied 56 in Form einer in sich geschlossenen, in der Nut 22 gehaltenen Schraubenfeder 58 aufweist. Das Vorspannglied 56 stützt sich einerseits im Wesentlichen am Nutboden 38 und andererseits an der Anlagefläche 52 ab. Die Schraubenfeder 58 ist schräg bezogen auf eine von ihr definierte Schraubenlinie 60 gewickelt, so dass einzelne Windungen 62 der Schraubenfeder 58 nicht im Wesentlichen in einer Ebene senkrecht zur Schraubenlinie 60 verlaufen, sondern relativ zu dieser bereits in einer Grundstellung der Schraubenfeder 58, in welcher keine äußeren Kräfte auf diese einwirken, etwa um 45° geneigt sind. Dies ist schematisch in Figur 6 dargestellt.

Das Vorspannglied 56 definiert eine Vorspanngliedlängslinie 64, die der Schraubenlinie 60 entspricht. Somit übt das Vorspannglied 56 eine quer, insbesondere senkrecht, zur Vorspanngliedlängslinie 64 wirkende Kraft auf den Behälteroberteildichtabschnitt 24 sowie den Behälterunterteildichtabschnitt 28 aus. Man kann auch sagen, dass das Vorspannglied 56 mit einer quer, insbesondere senkrecht, zur Vorspanngliedlängslinie 64 orientierten Außenfläche 66 über die Anlagefläche 52 indirekt am Behälteroberteildichtabschnitt 24 sowie am Behälterunterteildichtabschnitt 28 anliegt.

Die Schraubenfeder 58 ist einstückig und in sich geschlossen ausgebildet, so dass also nur ein einziges Vorspannglied 56 vorgesehen ist. Denkbar wäre es, auch zwei oder mehr Vorspannglieder 56 in Form von Schraubenfedern 58 vorzusehen, deren freie Enden miteinander verbunden sind.

Die in den Figuren 2 und 3 schematisch dargestellte Vorspanneinrichtung 54 ist teilweise vom Dichtelement 20 bedeckt oder umgeben. Bei der in den Figuren 2 und 3 schematisch dargestellten Variante einer Dichteinrichtung 18 ist der Behälteroberteildichtabschnitt 24 fest, aber beweglich mit dem Behälteroberteil 14 verbunden. Alternativ könnte die Anordnung auch umgekehrt ausgebildet sein, indem der Behälterunterteildichtabschnitt fest, aber beweglich mit dem Behälterunterteil 12 verbunden ist.

Ferner ist es auch denkbar, den Behälterunterteildichtabschnitt 28 unlösbar mit dem Behälterunterteil 12 zu verbinden. Alternativ kann auch der Behälteroberteildichtabschnitt 24 unlösbar mit dem Behälteroberteil 14 verbunden sein. Insgesamt ist die Vorspanneinrichtung 54 somit angeordnet zum Ausüben einer vorspannenden Kraft auf den Behälterunterteildichtabschnitt 28 und den Behälteroberteildichtabschnitt 24.

Der Behälterunterteildichtabschnitt 28 und der Behälterunterteil 12 sind relativ zueinander bewegbar angeordnet. Insbesondere kann der Behälterunterteil 12 ganz vom Behälterunterteildichtabschnitt 28 gelöst werden. Ebenso ist der Behälteroberteildichtabschnitt relativ zum Behälteroberteil 14 beweglich angeordnet.

Wird der Behälteroberteil 14, wie in Figur 3 schematisch dargestellt, auf den Behälterunterteil 112 aufgesetzt, treten zunächst die Längskante 46 und der Behälterunterteildichtflächenbereich 30 miteinander in Kontakt. Wird der Behälteroberteil 14 weiter in Richtung auf den Behälterunterteil 12 hin geschoben, wird die Schraubenfeder 58 senkrecht zu ihrer Schraubenlinie 60 vorgespannt. Dies äußert sich dadurch, dass die Windungen 62 sich noch weiter relativ zur Schraubenlinie 60 neigen, wie dies in Figur 7, und bei weiter fortschreitendem Druck auf die Schraubenfeder 58, in Figur 8 schematisch dargestellt ist. Eine maximale Neigung der Windungen 62 ist erreicht, wenn diese, wie in Figur 8 schematisch dargestellt, flächig aneinander anliegen.

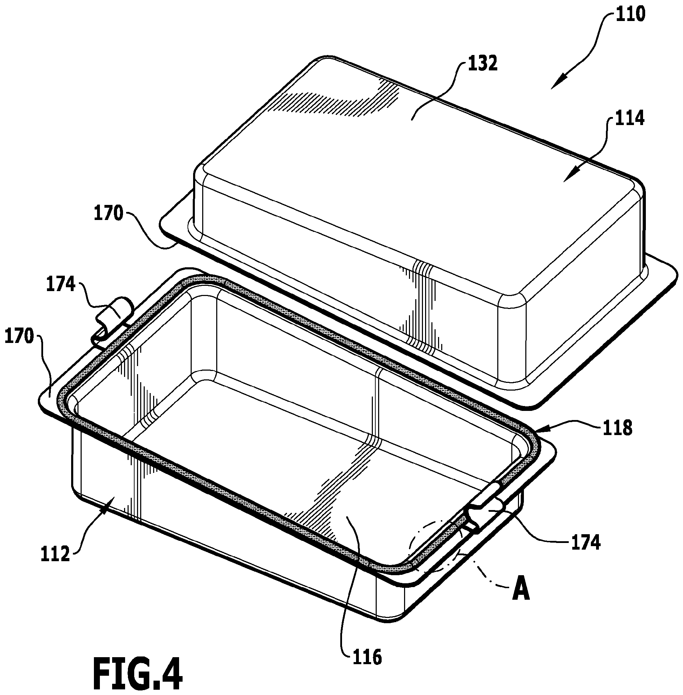

Schematisch ist in Figur 4 eine alternative Ausführungsform eines Sterilbehälters insgesamt mit dem Bezugszeichen 110 versehen. Er umfasst ein wannenförmiges Behälterunterteil 112 und ein im Wesentlichen identisch ausgebildetes Behälteroberteil 114 zum Verschließen des Behälterunterteils 112. Sie definieren wiederum in einer Schließstellung einen Behälterinnenraum 116. Eine Dichtfläche 170 des Behälterunterteils 112, ebenso wie eine Dichtfläche 170 des Behälteroberteils 113, definiert eine Ebene, die relativ zu einer von der Deckelwand 132 definierten Ebene etwas geneigt ist.

Auf der Dichtfläche 170 des Behälterunterteils 112 ist eine Dichteinrichtung 118 gehalten. Sie umfasst ein in sich geschlossenes, schlauchförmiges Dichtelement 120, welches eine umlaufende, hohle und einen Hohlraum 176 definierende Vorspanneinrichtungsaufnahme 172 definiert, in welcher eine Vorspanneinrichtung 154 umfassend ein eine Vorspanngliedlängslinie 164 definierendes Vorspannglied 156 in Form einer eine Schraubenlinie 160 definierenden Schraubenfeder 158 gehalten ist. Das Dichtelement 120 umhüllt somit die Vorspanneinrichtung 154 vollständig. Alternativ wäre es auch denkbar das Dichtelement 120 in Form eines Profilstranges ähnlich dem Dichtelement 120 auszubilden, welcher Profilstrang zwar eine schlauchförmige Grundform, jedoch eine umlaufende, seitliche Öffnung zur Vorspanneinrichtungsaufnahme 172 aufweist, durch welche Öffnung das Vorspannglied 156 in den Hohlraum 176 eingesetzt werden kann.

Das Dichtelement 120 kann beispielsweise an die Dichtfläche 170 angeklebt oder angeschweißt sein. Es ist damit fest mit dem Behälterunterteil 112 verbunden und hält die Vorspanneinrichtung 154 in definierter Weise in Position. Alternativ wäre es auch möglich, die Dichteinrichtung 118 in analoger Weise am Behälteroberteil 114 anzuordnen und festzulegen.

Zum keimdichten Verschließen des Sterilbehälters 110 wird der Behälteroberteil 114 mit seiner Dichtfläche 170 auf das Dichtelement 120 aufgesetzt. Dadurch wird ein Behälteroberteildichtabschnitt 124, welcher einen Behälteroberteildichtflächenbereich 126 definiert, gegen eine Außenfläche 166 der Schraubenfeder 158 gedrückt. In analoger Weise wird die Außenfläche 166 auch gegen einen Behälterunterteildichtabschnitt 128, welcher einen Behälterunterteildichtflächenbereich 130 definiert, gedrückt.

An Stirnseiten des Behälterunterteils 112 angeordnete klammerförmige Verschlusselemente 174 ermöglichen es, das Behälteroberteil 114 mit einer teilweise ausgelenkten Vorspanneinrichtung 154 in der Schließstellung zu halten. Die Verformung der Schraubenfeder 158 beim Schließen des Sterilbehälters 110 erfolgt analog der in den Figuren 6 bis 8 dargestellten Verformung der Schraubenfeder 58. Die Schraubenfeder 158 stellt sicher, dass der Behälteroberteildichtflächenbereich 126 und der Behälterunterteildichtflächenbereich 130 dauerhaft und sicher an den Dichtflächen 170 des Behälterunterteils 112 und der Behälteroberteils 114 anliegen.

Die Schraubenfedern 58 und 158 sind vorzugsweise aus einem Metall hergestellt, wobei sich insbesondere Legierungen aus Kupfer und Beryllium besonders gut eignen. Die Dichtelemente 20 und 120 sind aus einem Kunststoff hergestellt, beispielsweise durch Spritzgießen oder Extrudieren. Durch das Vorsehen der Vorspanneinrichtung 54 beziehungsweise 154 muss der Kunststoff, insbesondere auch dauerhaft, keine großen elastischen Eigenschaften aufweisen, so dass insbesondere auch Polytetrafluorethylen als Material zur Herstellung der Dichtelemente 20 und 120 geeignet ist. Optional können die Dichtelemente 20 und 120 auch aus einem Elastomer hergestellt sein oder einen solchen enthalten, beispielsweise Gummi.

Selbstverständlich sind weitere alternative Ausgestaltungen von Sterilbehältern mit entsprechenden Dichteinrichtungen, die Vorspanneinrichtungen umfassen, denkbar. Die besonderen Eigenschaften, insbesondere der Dichtelemente 20 und 120, werden im Wesentlichen dadurch erreicht, dass eine vorspannende Wirkung der Vorspannglieder 56 und 156 quer zu einer von ihnen definierten Vorspanngliedlängslinie 64 beziehungsweise 164 erfolgt und nicht, wie bei Schraubenfedern üblich, parallel zu den von diesen definierten Schraubenlinien.

Erforderliche Vorspannkräfte können in gezielter Weise eingestellt werden durch Gestaltung der Vorspannglieder 56 und 156, insbesondere durch Auswahl geeigneter Materialien sowie entsprechende Dimensionierung beispielsweise eines Durchmessers der Schraubenfeder 58 und 158 sowie des zur Wicklung derselben benutzten Drahtmaterials.

## Patentansprüche

1. Behälterunterteil (12; 112) eines Sterilbehälters (10; 110), welcher mit einem Behälteroberteil (14; 114) des Sterilbehälters (10; 110) verschließbar ist, welcher Behälterunterteil (12; 112) und welcher Behälteroberteil (14; 114) in einer Schließstellung einen Behälterinnenraum (16; 116) definieren, an welchem Behälterunterteil (12; 112) eine Dichteinrichtung (18; 118) zum gas- oder keimdichten Abdichten des Sterilbehälters (10; 110) in der Schließstellung gehalten ist, welche ein umlaufendes, am Behälterunterteil (12; 112) anliegendes und am Behälteroberteil (14; 114) anlegbares Dichtelement (20; 120) umfasst, wobei das Dichtelement (20; 120) einen Behälterunterteildichtabschnitt (28; 128) mit einem am Behälterunterteil (12; 112) anliegenden Behälterunterteildichtflächenbereich (30; 130) und einen Behälteroberteildichtabschnitt (24; 124) mit einem am Behälteroberteil (14; 114) anlegbaren Behälteroberteildichtflächenbereich (26; 126) umfasst, wobei die Dichteinrichtung (18; 118) eine Vorspanneinrichtung (54; 154) umfasst, welche eine vorspannende Kraft auf mindestens den Behälteroberteildichtabschnitt (24; 124) in Richtung vom Behälterunterteil (12; 112) weg ausübt, wobei die Vorspanneinrichtung (54; 154) mindestens ein Vorspannglied (56; 156) umfasst, welches eine vorspannende Kraft auf mindestens den Behälterunterteildichtabschnitt (28; 128) in Richtung auf den Behälterunterteil (12; 112) oder den Behälteroberteildichtabschnitt (24; 124) in Richtung auf den Behälteroberteil (14; 114) ausübt, wobei das mindestens eine Vorspannglied (56; 156) eine Schraubenfeder (58; 158) umfasst oder in Form einer Schraubenfeder (58; 158) ausgebildet ist, **dadurch gekennzeichnet, dass** die Schraubenfeder (58; 158) eine umlaufende Schraubenlinie (60; 160) definiert und wobei die Schraubenfeder (58; 158) eine quer, insbesondere senkrecht, zur Schraubenlinie (60; 160) wirkende Kraft auf den Behälteroberteildichtabschnitt (24; 124) und/oder den Behälterunterteildichtabschnitt (28; 128) ausübt.

2. Behälteroberteil (14; 114) eines Sterilbehälters (10; 110), welcher Behälteroberteil (14; 114) ausgebildet ist zum Verschließen eines Behälterunterteils (12; 112) des Sterilbehälters (10; 110), welcher Behälteroberteil (14; 114) und welcher Behälterunterteil (12; 112) in einer Schließstellung einen Behälterinnenraum (16; 116) definieren, an welchem Behälteroberteil (14; 114) eine Dichteinrichtung (18; 118) zum gas- oder keimdichten Abdichten des Sterilbehälters (10; 110) in der Schließstellung gehalten ist, welche ein umlaufendes, am Behälteroberteil (14; 114) anliegendes und am Behälterunterteil (12; 112) anlegbares Dichtelement (20; 120) umfasst, wobei das Dichtelement (20; 120) einen Behälteroberteildichtabschnitt (24; 124) mit einem am Behälteroberteil (14; 114) anliegenden Behälteroberteildichtflächenbereich (26; 126) und einen Behälterunterteildichtabschnitt (28; 128) mit einem am Behälterunterteil (12; 112) anlegbaren Behälterunterteildichtflächenbereich (30; 130) umfasst, wobei die Dichteinrichtung (18; 118) eine Vorspanneinrichtung (54; 154) umfasst, welche eine vorspannende Kraft auf mindestens den Behälterunterteildichtabschnitt (28; 128) in Richtung vom Behälteroberteil (14; 114) weg ausübt, wobei die Vorspanneinrichtung (54; 154) mindestens ein Vorspannglied (56; 156) umfasst, welches eine vorspannende Kraft auf mindestens den Behälterunterteildichtabschnitt (28; 128) in Richtung auf den Behälterunterteil (12; 112) oder den Behälteroberteildichtabschnitt (24; 124) in Richtung auf den Behälteroberteil (14; 114) ausübt, wobei das mindestens eine Vorspannglied (56; 156) eine Schraubenfeder (58; 158) umfasst oder in Form einer Schraubenfeder (58; 158) ausgebildet ist, **dadurch gekennzeichnet, dass** die Schraubenfeder (58; 158) eine umlaufende Schraubenlinie (60; 160) definiert und wobei die Schraubenfeder (58; 158) eine quer, insbesondere senkrecht, zur Schraubenlinie (60; 160) wirkende Kraft auf den Behälteroberteildichtabschnitt (24; 124) und/oder den Behälterunterteildichtabschnitt (28; 128) ausübt.

3. Sterilbehälter (10; 110) mit einem Behälterunterteil (12; 112) und einem Behälteroberteil (14; 114) zum Verschließen des Behälterunterteils (12; 112), welcher Behälterunterteil (12; 112) und welcher Behälteroberteil (14; 114) in einer Schließstellung einen Behälterinnenraum (16; 116) definieren, wobei eine Dichteinrichtung (18; 118) zum gas- oder keimdichten Abdichten des Sterilbehälters (10; 110) in der Schließstellung vorgesehen ist, welche ein umlaufendes und sowohl am Behälterunterteil (12; 112) als auch am Behälteroberteil (14; 114) in der Schließstellung anliegendes Dichtelement (20; 120) umfasst, wobei das Dichtelement (20; 120) einen Behälteroberteildichtabschnitt (24; 124) mit einem am Behälteroberteil (14; 114) anliegenden Behälteroberteildichtflächenbereich (26; 126) und einen Behälterunterteildichtabschnitt (28; 128) mit einem am Behälterunterteil (12; 112) anliegenden Behälterunterteildichtflächenbereich (30; 130) umfasst, wobei die Dichteinrichtung (18; 118) eine Vorspanneinrichtung (54; 154) umfasst, welche eine vorspannende Kraft auf mindestens den Behälterunterteildichtabschnitt (28; 128) in Richtung auf den Behälterunterteil (12; 112) oder den Behälteroberteildichtabschnitt (24; 124) in Richtung auf den Behälteroberteil (14; 114) ausübt, wobei die Vorspanneinrichtung (54; 154) mindestens ein Vorspannglied (56; 156) umfasst, welches eine vorspannende Kraft auf mindestens den Behälterunterteildichtabschnitt (28; 128) in Richtung auf den Behälterunterteil (12; 112) oder den Behälteroberteildichtabschnitt (24; 124) in Richtung auf den Behälteroberteil (14; 114) ausübt, wobei das mindestens eine Vorspannglied (56; 156) eine Schraubenfeder (58; 158) umfasst oder in Form einer Schraubenfeder (58; 158) ausgebildet ist, **dadurch gekennzeichnet, dass** die Schraubenfeder (58; 158) eine umlaufende Schraubenlinie (60; 160) definiert und wobei die Schraubenfeder (58; 158) eine quer, insbesondere senkrecht, zur Schraubenlinie (60; 160) wirkende Kraft auf den Behälteroberteildichtabschnitt (24; 124) und/oder den Behälterunterteildichtabschnitt (28; 128) ausübt.

4. Behälterunterteil, Behälteroberteil oder Sterilbehälter nach einem der voranstehenden Ansprüche, **dadurch gekennzeichnet, dass** die Vorspanneinrichtung (54; 154) angeordnet ist zum Ausüben einer vorspannenden Kraft auf den Behälterunterteildichtabschnitt (28; 128) und den Behälteroberteildichtabschnitt (24; 124).

5. Behälterunterteil oder Sterilbehälter nach einem der Ansprüche 1, 3 oder 4 soweit Anspruch 4 auf die Ansprüche 1 oder 3 rückbezogen ist, **dadurch gekennzeichnet, dass** der Behälterunterteildichtabschnitt (28; 128) und der Behälterunterteil (12; 112) relativ zueinander bewegbar angeordnet sind.

6. Behälteroberteil oder Sterilbehälter nach einem der Ansprüche 2, 3 oder 4 soweit Anspruch 4 auf die Ansprüche 2 oder 3 rückbezogen ist, **dadurch gekennzeichnet, dass** der Behälteroberteildichtabschnitt (24; 124) und der Behälteroberteil (14; 114) relativ zueinander bewegbar angeordnet sind.

7. Behälterunterteil oder Sterilbehälter nach einem der Ansprüche 1, 3, 4 soweit auf die Ansprüche 1 oder 3 rückbezogen oder 5, **dadurch gekennzeichnet, dass** der Behälterunterteildichtabschnitt (128) unlösbar mit dem Behälterunterteil (112) verbunden ist.

8. Behälteroberteil oder Sterilbehälter nach einem der Ansprüche 2, 3, 4 soweit auf die Ansprüche 2 oder 3 rückbezogen oder 6, **dadurch gekennzeichnet, dass** der Behälteroberteildichtabschnitt (24) unlösbar mit dem Behälteroberteil (14) verbunden ist.

9. Behälterunterteil, Behälteroberteil oder Sterilbehälter nach einem der voranstehenden Ansprüche, **dadurch gekennzeichnet, dass** die Vorspanneinrichtung (54; 154) mindestens teilweise vom Dichtelement (20; 120) bedeckt oder umgeben ist.

10. Behälterunterteil, Behälteroberteil oder Sterilbehälter nach einem der voranstehenden Ansprüche, **dadurch gekennzeichnet, dass** das Dichtelement (120) die Vorspanneinrichtung (154) vollständig umhüllt.

11. Behälterunterteil, Behälteroberteil oder Sterilbehälter nach einem der voranstehenden Ansprüche, **dadurch gekennzeichnet, dass** das mindestens eine Vorspannglied (56; 156) eine Vorspanngliedlängslinie (64; 164) definiert und dass das mindestens eine Vorspannglied (56; 156) mit einer quer, insbesondere senkrecht, zur Vorspanngliedlängslinie (64; 164) orientierten Außenfläche (66; 166) am Behälteroberteildichtabschnitt (24; 124) und/oder am Behälterunterteildichtabschnitt (28; 128) anliegt.

12. Behälterunterteil, Behälteroberteil oder Sterilbehälter nach einem der voranstehenden Ansprüche, **dadurch gekennzeichnet, dass** das mindestens eine Vorspannglied (56; 156) eine Vorspanngliedlängslinie (64; 164) definiert und dass das mindestens eine Vorspannglied (56; 156) eine quer, insbesondere senkrecht, zur Vorspanngliedlängslinie (64; 164) wirkende Kraft auf den Behälteroberteildichtabschnitt (24; 124) und/oder den Behälterunterteildichtabschnitt (28; 128) ausübt.

13. Behälterunterteil, Behälteroberteil oder Sterilbehälter nach einem der voranstehenden Ansprüche, **dadurch gekennzeichnet, dass** das Dichtelement (120) eine Vorspanneinrichtungsaufnahme (172) zum mindestens teilweisen Aufnehmen der Vorspanneinrichtung (154) umfasst.

14. Behälterunterteil, Behälteroberteil oder Sterilbehälter nach Anspruch 13, **dadurch gekennzeichnet, dass** die Vorspanneinrichtungsaufnahme (172) umlaufend geöffnet ist oder dass die Vorspanneinrichtungsaufnahme (172) geschlossen ist.

15. Behälterunterteil, Behälteroberteil oder Sterilbehälter nach einem der voranstehenden Ansprüche, **dadurch gekennzeichnet, dass** die Vorspanneinrichtung (54; 154) am Dichtelement (20; 120) gehalten ist.

## Claims

1. Lower container part (12; 112) of a sterile container (10; 110), said lower container part being closable by an upper container part (14; 114) of the sterile container (10; 110), said lower container part (12; 112) and said upper container part (14; 114) defining, in a closed position, a container interior (16; 116), a sealing device (18; 118) being held on said lower container part (12; 112) for the gastight or germ-proof sealing of the sterile container (10; 110) in the closed position, said sealing device comprising a circumferential sealing element (20; 120) bearing against the lower container part (12; 112) and adapted to be placed against the upper container part (14; 114), said sealing element (20; 120) comprising a lower container part sealing section (28; 128) with a lower container part sealing surface region (30; 130) bearing against the lower container part (12; 112), and an upper container part sealing section (24; 124) with an upper container part sealing surface region (26; 126) adapted to be placed against the upper container part (14; 114), wherein said sealing device comprises a pretensioning device (54; 154), which exerts a pretensioning force on at least the upper container part sealing section (24; 124) in the direction away from the lower container part (12; 112), wherein the pretensioning device (54; 154) comprises at least one pretensioning member (56; 156), which exerts a pretensioning force on at least the lower container part sealing section (28; 128) in the direction towards the lower container part (12; 112) or on the upper container part sealing section (24; 124) in the direction towards the upper container part (14; 114), wherein the at least one pretensioning member (56; 156) comprises a helical spring (58; 158) or is configured in the form of a helical spring (58; 158), **characterized in that** the helical spring (58; 158) defines an all-round helical line (60; 160), and wherein the helical spring (58; 158) exerts a force acting transversely, in particular, perpendicularly, to the helical line (60; 160) on at least one of the upper container part sealing section (24; 124) and/or the lower container part sealing section (28; 128).

2. Upper container part (14; 114) of a sterile container (10; 110), said upper container part (14; 114) being designed to close a lower container part of the sterile container (12; 112), said upper container part (14; 114) and said lower container part (12; 112) defining, in a closed position, a container interior (16; 116), a sealing device (18; 118) being held on said upper container part (14; 114) for the gastight or germ-proof sealing of the sterile container (10; 110), in the closed position, said sealing device (18; 118) comprising a circumferential sealing element (20; 120) bearing against the upper container part (14; 114) and adapted to be placed against the lower container part (12; 112), said sealing element (20; 120) comprising an upper container part sealing section (24; 124) with an upper container part sealing surface region (26; 126) bearing against the upper container part (14; 114), and a lower container part sealing section (28; 128) with a lower container part sealing surface region (30; 130) adapted to be placed against the lower container part (12; 112), wherein said sealing device (18; 118) comprises a pretensioning device (54; 154), which exerts a pretensioning force on at least the lower container part sealing section (28; 128) in the direction away from the upper container part (14; 114), wherein the pretensioning device (54; 154) comprises at least one pretensioning member (56; 156), which exerts a pretensioning force on at least the lower container part sealing section (28; 128) in the direction towards the lower container part (12; 112) or on the upper container part sealing section (24; 124) in the direction towards the upper container part (14; 114), wherein the at least one pretensioning member (56; 156) comprises a helical spring (58; 158) or is configured in the form of a helical spring (58; 158) **characterized in that** the helical spring defines an all-round helical line (60; 160), and wherein the helical spring (58; 158) exerts a force acting transversely, in particular, perpendicularly, to the helical line (60; 160) on the upper container part sealing section (24; 124) and/or the lower container part sealing section (28; 128).

3. Sterile container (10; 110) having a lower container part (12; 112) and an upper container part (14; 114) for closing the lower container part (12; 112), said lower container part (12; 112) and said upper container part (14; 114) defining, in a closed position, a container interior (16; 116), wherein a sealing device (18; 118) is provided for the gastight or germ-proof sealing of the sterile container (10; 110) in the closed position, said sealing device (18; 118) comprising a circumferential sealing element (20; 120) bearing against both the lower container part (12; 112) and the upper container part (14; 114) in the closed position, wherein said sealing element (20; 120) comprises an upper container part sealing section (24; 124) with an upper container part sealing surface region (26; 126) bearing against the upper container part (14; 114), and a lower container part sealing section (28; 128) with a lower container part sealing surface region (30; 130) bearing against the lower container part (12; 112), wherein the sealing device (18; 118) comprises a pretensioning device (54; 154), which exerts a pretensioning force on at least the lower container part sealing section (28; 128) in the direction towards the lower container part (12; 112) or on the upper container part sealing section (24; 124) in the direction towards the upper container part (14; 114), wherein the pretensioning device (54; 154) comprises at least one pretensioning member (56; 156), which exerts a pretensioning force on at least the lower container part sealing section (28; 128) in the direction towards the lower container part (12; 112) or on the upper container part sealing section (24; 124) in the direction towards the upper container part (14; 114), wherein the at least one pretensioning member (56; 156) comprises a helical spring (58; 158) or is configured in the form of a helical spring (58; 158) **characterized in that** the helical spring defines an all-round helical line (60; 160), and wherein the helical spring (58; 158) exerts a force acting transversely, in particular, perpendicularly, to the helical line (60; 160) on the upper container part sealing section (24; 124) and/or the lower container part sealing section (28; 128).

4. Lower container part, upper container part or sterile container in accordance with any one of the preceding claims, **characterized in that** the pretensioning device (54; 154) is arranged so as to exert a pretensioning force on the lower container part sealing section (28; 128) and on the upper container part sealing section (24; 124).

5. Lower container part or sterile container in accordance with any one of claims 1, 3 or 4 insofar as claim 4 is appended to claim 1 or 3, **characterized in that** the lower container part sealing section (28; 128) and the lower container part (12; 112) are arranged for movement relative to each other.

6. Upper container part or sterile container in accordance with any one of claims 2, 3 or 4 insofar as claim 4 is appended to claim 2 or 3, **characterized in that** the upper container part sealing section (24; 124) and the upper container part (14; 114) are arranged for movement relative to each other.

7. Lower container part or sterile container in accordance with any one of claims 1, 3, 4 as appended to claim 1 or 3, or 5, **characterized in that** the lower container part sealing section (128) is undetachably connected to the lower container part (112).

8. Upper container part or sterile container in accordance with any one of claims 2, 3, 4 as appended to claim 2 or 3, or 6, **characterized in that** the upper container part sealing section (24) is undetachably connected to the upper container part (14).

9. Lower container part, upper container part or sterile container in accordance with any one of the preceding claims, **characterized in that** the pretensioning device (54; 154) is at least partly covered or surrounded by the sealing element (20; 120).

10. Lower container part, upper container part or sterile container in accordance with any one of the preceding claims, **characterized in that** the sealing element (120) completely encloses the pretensioning device (154).

11. Lower container part, upper container part or sterile container in accordance with any one of the preceding claims, **characterized in that** the at least one pretensioning member (56; 156) defines a pretensioning member longitudinal line (64; 164), and **in that** the at least one pretensioning member (56; 156) bears with an outer surface (66; 166), oriented transversely, in particular, perpendicularly, to the pretensioning member longitudinal line (64; 164), against the upper container part sealing section (24; 124) and/or the lower container part sealing section (28; 128).

12. Lower container part, upper container part or sterile container in accordance with any one of the preceding claims, **characterized in that** the at least one pretensioning member (56; 156) defines a pretensioning member longitudinal line (64; 164), and **in that** the at least one pretensioning member (56; 156) exerts a force acting transversely, in particular, perpendicularly, to the pretensioning member longitudinal line (64; 164) on the upper container part sealing section (24; 124) and/or the lower container part sealing section (28; 128).

13. Lower container part, upper container part or sterile container in accordance with any one of the preceding claims, **characterized in that** the sealing element (120) comprises a pretensioning device receptacle (172) for at least partly receiving the pretensioning device (154).

14. Lower container part, upper container part or sterile container in accordance with claim 13, **characterized in that** the pretensioning device receptacle (172) is open circumferentially or **in that** the pretensioning device receptacle (172) is closed.

15. Lower container part, upper container part or sterile container in accordance with any one of the preceding claims, **characterized in that** the pretensioning device (54; 154) is held on the sealing element (20; 120).

## Revendications

1. Pièce inférieure de récipient (12 ; 112) d'un récipient stérile (10 ; 110), lequel peut être fermé avec une pièce supérieure de récipient (14 ; 114) du récipient stérile (10 ; 110), laquelle pièce inférieure de récipient (12 ; 112) et laquelle pièce supérieure de récipient (14 ; 114) définissent dans une position fermée une chambre intérieure de récipient (16 ; 116), contre laquelle pièce inférieure de récipient (12 ; 112) est maintenue un dispositif d'étanchéité (18 ; 118) pour rendre le récipient stérile (10; 110) étanche aux gaz ou aux germes dans la position fermée, lequel comprend un élément d'étanchéité (20 ; 120) périphérique appliqué contre la pièce inférieure de récipient (12 ; 112) et applicable contre la pièce supérieure de récipient (14; 114), dans lequel l'élément d'étanchéité (20 ; 120) comprend une partie d'étanchéité de pièce inférieure de récipient (28; 128) avec une zone de surface d'étanchéité de pièce inférieure de récipient (30 ; 130) appliquée contre la pièce inférieure de récipient (12 ; 112) et une partie d'étanchéité de pièce supérieure de récipient (24 ; 124) avec une zone de surface d'étanchéité de pièce supérieure de récipient (26 ; 126) applicable contre la pièce supérieure de récipient (14; 114), dans lequel le dispositif d'étanchéité (18 ; 118) comprend un dispositif de précontrainte (54 ; 154), lequel exerce une force de précontrainte sur au moins la partie d'étanchéité de pièce supérieure de récipient (24 ; 124) dans une direction s'éloignant de la pièce inférieure de récipient (12 ; 112), dans lequel le dispositif de précontrainte (54 ; 154) comprend au moins un membre de précontrainte (56 ; 156), lequel exerce une force de précontrainte sur au moins la partie d'étanchéité de pièce inférieure de récipient (28 ; 128) dans la direction de la pièce inférieure de récipient (12; 112) ou sur la partie d'étanchéité de pièce supérieure de récipient (24 ; 124) dans la direction de la pièce supérieure de récipient (14 ; 114), dans lequel le au moins un membre de précontrainte (56 ; 156) comprend un ressort hélicoïdal (58 ; 158) ou est réalisé sous la forme d'un ressort hélicoïdal (58; 158), **caractérisé en ce que** le ressort hélicoïdal (58; 158) définit une hélice (60 ; 160) périphérique et dans lequel le ressort hélicoïdal (58 ; 158) exerce une force agissant transversalement, en particulier perpendiculairement, à l'hélice (60; 160) sur la partie d'étanchéité de pièce supérieure de récipient (24 ; 124) et/ou sur la partie d'étanchéité de pièce inférieure de récipient (28 ; 128).

2. Pièce supérieure de récipient (14 ; 114) d'un récipient stérile (10 ; 110), laquelle pièce supérieure de récipient (14; 114) est formée pour fermer une pièce inférieure de récipient (12 ; 112) du récipient stérile (10 ; 110), laquelle pièce supérieure de récipient (14; 114) et laquelle pièce inférieure de récipient (12 ; 112) définissent dans une position fermée une chambre intérieure de récipient (16; 116), contre laquelle pièce supérieure de récipient (14; 114) est maintenu un dispositif d'étanchéité (18 ; 118) pour rendre le récipient stérile (10 ; 110) étanche aux gaz ou aux germes dans la position fermée, lequel comprend un élément d'étanchéité (20; 120) périphérique appliqué contre la pièce supérieure de récipient (14 ; 114) et applicable contre la pièce inférieure de récipient (12; 112), dans lequel l'élément d'étanchéité (20 ; 120) comprend une partie d'étanchéité de pièce supérieure de récipient (24; 124) avec une zone de surface d'étanchéité de pièce supérieure de récipient (26 ; 126) appliquée contre la pièce supérieure de récipient (14 ; 114) et une partie d'étanchéité de pièce inférieure de récipient (28; 128) avec une zone de surface d'étanchéité de pièce inférieure de récipient (30 ; 130) applicable contre la pièce inférieure de récipient (12; 112), dans lequel le dispositif d'étanchéité (18 ; 118) comprend un dispositif de précontrainte (54 ; 154), lequel exerce une force de précontrainte sur au moins la partie d'étanchéité de pièce inférieure de récipient (28 ; 128) dans une direction s'éloignant de la pièce supérieure de récipient (14 ; 114), dans lequel le dispositif de précontrainte (54 ; 154) comprend au moins un membre de précontrainte (56 ; 156), lequel exerce une force de précontrainte sur au moins la partie d'étanchéité de pièce inférieure de récipient (28 ; 128) dans la direction de la pièce inférieure de récipient (12 ; 112) ou sur la partie d'étanchéité de pièce supérieure de récipient (24 ; 124) dans la direction de la pièce supérieure de récipient (14 ; 114), dans lequel le au moins un membre de précontrainte (56 ; 156) comprend un ressort hélicoïdal (58 ; 158) ou est réalisé sous la forme d'un ressort hélicoïdal (58; 158), **caractérisé en ce que** le ressort hélicoïdal (58; 158) définit une hélice (60 ; 160) périphérique et dans lequel le ressort hélicoïdal (58 ; 158) exerce une force agissant transversalement, en particulier perpendiculairement, à l'hélice (60; 160) sur la partie d'étanchéité de pièce supérieure de récipient (24 ; 124) et/ou sur la partie d'étanchéité de pièce inférieure de récipient (28 ; 128).

3. Récipient stérile (10 ; 110) avec une pièce inférieure de récipient (12 ; 112) et une pièce supérieure de récipient (14; 114) pour fermer la pièce inférieure de récipient (12; 112), laquelle pièce inférieure de récipient (12; 112) et laquelle pièce supérieure de récipient (14 ; 114) définissent dans une position fermée une chambre intérieure de récipient (16 ; 116), dans lequel est prévu un dispositif d'étanchéité (18 ; 118) pour rendre le récipient stérile (10 ; 110) étanche aux gaz ou aux germes dans la position fermée, lequel comprend un élément d'étanchéité (20; 120) périphérique et appliqué tant contre la pièce inférieure de récipient (12; 112) que contre la pièce supérieure de récipient (14; 114) dans la position fermée, dans lequel l'élément d'étanchéité (20 ; 120) comprend une partie d'étanchéité de pièce supérieure de récipient (24 ; 124) avec une zone de surface d'étanchéité de pièce supérieure de récipient (26 ; 126) appliquée contre la pièce supérieure de récipient (14; 114) et une partie d'étanchéité de pièce inférieure de récipient (28 ; 128) avec une zone de surface d'étanchéité de pièce inférieure de récipient (30; 130) appliquée contre la pièce inférieure de récipient (12 ; 112), dans lequel le dispositif d'étanchéité (18 ; 118) comprend un dispositif de précontrainte (54; 154), lequel exerce une force de précontrainte sur au moins la partie d'étanchéité de pièce inférieure de récipient (28 ; 128) dans la direction de la pièce inférieure de récipient (12 ; 112) ou sur la partie d'étanchéité de pièce supérieure de récipient (24 ; 124) dans la direction de la pièce supérieure de récipient (14 ; 114), dans lequel le dispositif de précontrainte (54 ; 154) comprend au moins un membre de précontrainte (56 ; 156), lequel exerce une force de précontrainte sur au moins la partie d'étanchéité de pièce inférieure de récipient (28 ; 128) dans la direction de la pièce inférieure de récipient (12 ; 112) ou sur la partie d'étanchéité de pièce supérieure de récipient (24 ; 124) dans la direction de la pièce supérieure de récipient (14 ; 114), dans lequel le au moins un membre de précontrainte (56 ; 156) comprend un ressort hélicoïdal (58 ; 158) ou est réalisé sous la forme d'un ressort hélicoïdal (58; 158), **caractérisé en ce que** le ressort hélicoïdal (58; 158) définit une hélice (60 ; 160) périphérique et dans lequel le ressort hélicoïdal (58 ; 158) exerce une force agissant transversalement, en particulier perpendiculairement, à l'hélice (60; 160) sur la partie d'étanchéité de pièce supérieure de récipient (24 ; 124) et/ou sur la partie d'étanchéité de pièce inférieure de récipient (28 ; 128).

4. Pièce inférieure de récipient, pièce supérieure de récipient ou récipient stérile selon l'une quelconque des revendications précédentes, **caractérisés en ce que** le dispositif de précontrainte (54; 154) est agencé pour l'exercice d'une force de précontrainte sur la partie d'étanchéité de pièce inférieure de récipient (28 ; 128) et la partie d'étanchéité de pièce supérieure de récipient (24 ; 124) .

5. Pièce inférieure de récipient ou récipient stérile selon l'une des revendications 1, 3 ou 4 pour autant que la revendication 4 soit rattachée aux revendications 1 ou 3, **caractérisés en ce que** la partie d'étanchéité de pièce inférieure de récipient (28 ; 128) et la pièce inférieure de récipient (12 ; 112) sont agencées de manière déplaçable l'une par rapport à l'autre.

6. Pièce supérieure de récipient ou récipient stérile selon l'une des revendications 2, 3 ou 4 pour autant que la revendication 4 soit rattachée aux revendications 2 ou 3, **caractérisés en ce que** la partie d'étanchéité de pièce supérieure de récipient (24 ; 124) et la pièce supérieure de récipient (14 ; 114) sont agencées de manière déplaçable l'une par rapport à l'autre.

7. Pièce inférieure de récipient ou récipient stérile selon l'une des revendications 1, 3, 4 pour autant que rattachée aux revendications 1 ou 3, ou 5, **caractérisés en ce que** la partie d'étanchéité de pièce inférieure de récipient (128) est raccordée de manière inamovible à la pièce inférieure de récipient (112).

8. Pièce supérieure de récipient ou récipient stérile selon l'une des revendications 2, 3, 4 pour autant que rattachée aux revendications 2 ou 3, ou 6, **caractérisés en ce que** la partie d'étanchéité de pièce supérieure de récipient (24) est raccordée de manière inamovible à la pièce supérieure de récipient (14).

9. Pièce inférieure de récipient, pièce supérieure de récipient ou récipient stérile selon l'une quelconque des revendications précédentes, **caractérisés en ce que** le dispositif de précontrainte (54; 154) est au moins partiellement recouvert ou entouré par l'élément d'étanchéité (20; 120).

10. Pièce inférieure de récipient, pièce supérieure de récipient ou récipient stérile selon l'une quelconque des revendications précédentes, **caractérisés en ce que** l'élément d'étanchéité (120) enrobe complètement le dispositif de précontrainte (154).

11. Pièce inférieure de récipient, pièce supérieure de récipient ou récipient stérile selon l'une quelconque des revendications précédentes, **caractérisés en ce que** le au moins un membre de précontrainte (56; 156) définit une ligne longitudinale de membre de précontrainte (64 ; 164) et **en ce que** le au moins un membre de précontrainte (56; 156) s'applique avec une surface extérieure (66; 166) orientée transversalement, en particulier perpendiculairement, par rapport à la ligne longitudinale de membre de précontrainte (64 ; 164) contre la partie d'étanchéité de pièce supérieure de récipient (24 ; 124) et/ou contre la partie d'étanchéité de pièce inférieure de récipient (28 ; 128) .

12. Pièce inférieure de récipient, pièce supérieure de récipient ou récipient stérile selon l'une quelconque des revendications précédentes, **caractérisés en ce que** le au moins un membre de précontrainte (56; 156) définit une ligne longitudinale de membre de précontrainte (64 ; 164) et **en ce que** le au moins un membre de précontrainte (56 ; 156) exerce une force agissant transversalement, en particulier perpendiculairement, par rapport à la ligne longitudinale de membre de précontrainte (64 ; 164) sur la partie d'étanchéité de pièce supérieure de récipient (24; 124) et/ou sur la partie d'étanchéité de pièce inférieure de récipient (28 ; 128) .

13. Pièce inférieure de récipient, pièce supérieure de récipient ou récipient stérile selon l'une quelconque des revendications précédentes, **caractérisés en ce que** l'élément d'étanchéité (120) comprend un logement de dispositif de précontrainte (172) pour une réception au moins partielle du dispositif de précontrainte (154).

14. Pièce inférieure de récipient, pièce supérieure de récipient ou récipient stérile selon la revendication 13, **caractérisés en ce que** le logement de dispositif de précontrainte (172) est ouvert de manière périphérique ou **en ce que** le logement de dispositif de précontrainte (172) est fermé.

15. Pièce inférieure de récipient, pièce supérieure de récipient ou récipient stérile selon l'une quelconque des revendications précédentes, **caractérisés en ce que** le dispositif de précontrainte (54; 154) est maintenu contre l'élément d'étanchéité (20 ; 120).
